# EUROPEAN PATENT APPLICATION

(11) **EP 3 372 225 A1**
(43) Date of publication of application: **12.09.2018**
(21) Application number: 17160206.3
(22) Date of filing: 09.03.2017
(51) Int. Cl.: A61K 9/16

(54) **NOVEL DOSAGE FORM**

(71) Applicant: Develco Pharma Schweiz AG, 4133 Pratteln (CH)
(72) Inventor: Scheer, Mathias, 79664 Wehr (DE); Rey, Helene, 68680 Kembs (FR)
(74) Representative: CH Kilger Anwaltspartnerschaft mbB

(57) **Abstract**

The present invention relates to a solid oral pharmaceutical dosage form with a novel and well defined order of specific coatings allowing for the very defined and controlled release of at least one pharmaceutical active ingredient (API) and a broad range of potential uses.

Particularly, the dosage form according to the invention comprises an immediate release active core, a first delayed-release layer comprising an enteric coating, a second sustained release layer comprising a retard polymer, and a third immediate release layer comprising an active pharmaceutical ingredient. The solid oral pharmaceutical dosage form as such is characterized in having a bi-modal release profile of the at least one API with a much less variable second delayed release than known prior art pharmaceutical compositions, allowing for the application of different APIs or combinations thereof in for a variety of indications.

## Description

### FIELD OF INVENTION

The present invention is in the field of medicine, more in particular in the field of controlled release solid oral dosage forms for improved therapy. The dosage forms according to the invention can be used for different indications, depending on the active pharmaceutical ingredient(s) contained. For example, it can be used in the treatment of ADHD, pain, insomnia, gastroesophageal reflux diseases, allergies such as allergic rhinitis, high blood pressure, and diabetes, within others.

### BACKGROUND

Controlled release dosage forms are key for the development of improved therapies, both through improved patient compliance and decreased incidences of adverse drug reactions. Controlled dosage forms may be used to delay absorption of a pharmaceutical active ingredient (API) until it has reached certain portions of the alimentary tract. Sustained release (SR) formulations are aiming at providing longer periods of pharmacologic action compared to periods obtained after administration of immediate-release (IR) dosage forms. They are further used to maintain a desired API concentration in the blood stream for a longer period than it would occur when conventional IR dosage forms are administered. Such longer periods of response provide for many therapeutic benefits which cannot be achieved with corresponding short acting, IR preparations. For example, therapy can be continued without interrupting the sleep of the patient. This is particularly important in the treatment of a patient with mild to severe pain. Another advantage of longer acting drug preparations is the improved patient compliance resulting from the avoidance of missed doses through patient forgetfulness.

Unless conventional rapid acting medication carefully administered at frequent intervals, peaks and valleys in the blood level of the API may occur due to rapid absorption, metabolic inactivation and systemic excretion of the compound, generating problems in therapy maintenance of the patient. Thus, it is common goal in the field of pharmacologic research to develop sustained release dosage formulations that provide therapeutic concentrations of the API in blood which are maintained throughout the dosing interval, while the peak/trough concentration ratios are reduced. Hereby, drug development focusses on the many variables that influence the *in vivo* release and subsequent absorption of the active ingredients from the gastrointestinal tract.

Several prior art compositions exist which provide sustained release of pharmacologically active substances contained in the compositions after oral administration. These known sustained release formulations include specially matrix formulations, coated formulations with diffusion membrane, osmotic systems and ion exchange resins. Formulations may be in form of pellets, tablets, capsules. In these formulations, the slow release of the active medicament is brought about through selective porosity of the coating, through a hole, or through compounding with a special matrix to affect the release of a drug. Some sustained release formulations provide related sequential release of a single dose of an active compound at predetermined periods after administration.

Controlled release compositions have constituted a clear advance in the art, particularly for medication available for conditions such as Attention Deficit Hyperactivity Disorder (ADHD), for the treatment of insomnia, pain and others.

Attention Deficit Disorders is a common psychiatric disorder in children and characterized by inattention and impulsivity.

Stimulating agents, such as amphetamines, have been shown to be effective in the treatment of children with disorders of activity modulation and attention regulation. Methylphenidate {dl-threo-methyl-2-phenyl-2-(2-piperidyl) acetate} is the psychostimulant used most frequently in the treatment of hyperactivity and attention deficit disorder. It appears to have a higher incidence of positive effects and a lower incidence of adverse effects than other psychostimulants. The efficacy of methylphenidate in improving attention and behavioral symptoms was shown in several studies.

Because of their short half-life, immediate release methylphenidate preparations require frequent administration at short intervals to ensure adequate treatment, e.g. throughout a child's school day. The rapid onset and offset of immediate release methylphenidate preparations means that a medicated child with ADHD will be maximally affected only for relatively brief periods during the day. Due to its short half-life, methylphenidate is usually given twice per day, usually once after breakfast and once during the school day, an event that some children and some school personnel apparently avoid, resulting in poor compliance with prescribed regimens. It is possible that in part poor compliance in taking the medication may explain the variable and conflicting results reported in many studies of the effect of medication on improving the behavior of hyperactive children. These limitations of immediate release methylphenidate led to interest in products with longer effective periods of action.

Ritalin^{®} SR is one of the commercially available sustained release forms of methylphenidate. Several opinion leaders in treatment of ADHD however, have stated in respect to the results of clinical trials of Ritalin^{®} SR that it does not have a sufficiently early onset of effect to allow for behavioral management in the early morning. Said experts also state that Ritalin^{®} SR does not have the beneficial late effects that would be produced by a lunch time dose of immediate release methylphenidate, and thus defeating the purpose of using an SR formulation. Additionally, the effects of Ritalin^{®} SR are inconsistent or erratic over the course of the day.

Consequently, there is a need to develop drug bi-modal formulations which provide a rapid onset, and a prolonged action, followed by rapid offset of effect in order to overcome the deficiencies of the current state of the art.

WO 9903471 A1 refers to dosage forms for oral administration of methylphenidate. The dosage forms according to WO 9903471 A1 provide a substantially immediate dose of methylphenidate upon ingestion, followed by one or more additional doses at predetermined times. The corresponding dosage form described in WO 9903471 A1 contains two groups of particles, each containing the methylphenidate drug, wherein the first group of particles provides a substantially immediate dose and the second group of particles provides delayed release.

Several other prior art exist that discloses such multi-particulate modified release compositions. All of these dosage forms have in common that they are complex particle mixtures in which the particles have to be introduced into the tablet or encapsulated at a very defined ratio, leading to manufacturing variabilities and decreasing the patient safety.

Other prior art documents are directed to controlled release formulations comprising the active ingredient with sustained release layer coating. These systems avoid the manufacturing problems of multiple particle systems. Furthermore, and in contrast to conventional immediate release systems only comprising an enteric coating, systems with sustained release coating have the advantage that the active ingredient is not released at once after the enteric coating is dissolved. The disadvantage of such systems is however, that the sustained release coating slowly starts becoming permeable from the moment it is put in contact with water. This gradual increase in permeability is entirely pH independent and only depends on the amount of time the tablet has been in contact with water. The problem with a tablet with such a coating is that it slowly starts releasing the active ingredient from the moment the tablet is swallowed.

Further described in prior art are multi-layer release (MLR) systems, comprising an outer immediate release (IR) layer, a release delaying layer (i.e. due to enteric coating) coated over the sustained release layer, and an IR core. These systems also avoid the manufacturing problems of multiple particle systems and have the advantage that the active ingredient is not released at once after the enteric coating is dissolved, due to the sustained release layer below.

However, in these multi-layer systems the release of the active ingredient also only starts once the enteric coating is dissolved, i.e. once the tablet has reached a specific pH in the gut. The point at which such pH is attained depends on each individual person, e.g. on what the patient has consumed beforehand. Therefore, the time of release of the API is also not well defined in such controlled release compositions and there is high variation as to where and when the tablet starts releasing the active ingredient. Consequently, such compositions show high variability from patient to patient in the delayed release phase.

These problems clearly show that it is not trivial to develop suitable and safe controlled release systems. Albeit the diverse prior art available, there is still a need for improved drug delivery systems that show less variability from patient to patient while being easy to produce with low manufacturing variability and high patient compliance and safety. This need is satisfied by the novel dosage form according to the invention.

### SUMMARY

The present invention relates to a solid oral pharmaceutical dosage form with a novel and well defined order of specific coatings and containing at least one pharmaceutical active ingredient (API). Particularly, the dosage form according to the invention comprises an immediate release active core, a first delayed-release layer comprising an enteric coating, a second sustained release layer comprising a retard polymer, and a third immediate release layer comprising an active pharmaceutical ingredient. As such, the novel dosage form according to the invention is characterized in having a bi-modal release profile, with a much more defined and controlled second release than achieved with prior art pharmaceutical compositions.

### DETAILED DESCRIPTION OF INVENTION

The inventors surprisingly found that with the specific dosage form according to the invention, consisting of a novel and well defined order of specific coatings and comprising at least one pharmaceutical active ingredient, a very defined and controlled release of the at least one active pharmaceutical ingredient (API) can be achieved. This novel oral dosage form results in specific and beneficial pharmacokinetics that contribute to the benefit of the patient having to be treated with one or more APIs. The dosage form of the invention can be applied to a broad range of APIs and consequently has many indications.

Thus, in a first aspect, the invention relates to a solid oral pharmaceutical dosage form comprising at least one active pharmaceutical ingredient or a pharmaceutical acceptable salt, an isomeric form, a prodrug, or metabolite thereof, wherein the solid oral pharmaceutical dosage form comprises an immediate release active core, a first delayed-release layer comprising an enteric coating, a second sustained release layer comprising a retard polymer, and a third immediate release layer comprising an active pharmaceutical ingredient, and wherein the solid oral pharmaceutical dosage form exhibits a bi-modal release profile of the at least one active pharmaceutical ingredient.

The novel controlled release dosage form according to the invention as such is based on a series of layers with different characteristics - an outer immediate release layer comprising an API, a sustained release layer, a release delaying layer comprising and enteric polymer and an immediate release active core. This layered formulation is designed such that upon oral administration, the formulation provides a rapid dissolution and absorption of the outer layer of the formulation which contains a portion of the API in immediate release form, thereby resulting in a rapid rise of the drug to therapeutic plasma levels. This is followed by a period of controlled release of the drug from the formulation. The absorption of the main portion of the API is delayed until a point at which the sustained release layer is sufficiently permeable and a pH is attained for the enteric coating below to be dissolved. That way, the dosage form is able to mitigate the variability of the start of the release caused by the pH variability in the gut and provides for the particular and un-expected advantages over prior art.

Thus, the dosage form according to the invention is characterized in having a first immediate release of an API (IR release) and a later delayed second release (DR) of same API or another, resulting in the bi-modal release profile of the one or more APIs according to the invention.

Therefore, the novel dosage form can be used essentially for any therapy in which a first immediate release of the active ingredient is required or beneficial, and later a second, delayed release of the same or a different API is needed (e.g. for later provision of a drug during the day or while the patient is sleeping).

The second release of the API can be an immediate or sustained release. However, in any case, the beginning of the second release is very defined and much less variable than in other bi-modal release formulation in prior art pharmaceutical compositions. This advantage is achieved by the specific ordering of the layers. The inventors surprisingly found that when having a composition in which the sustained release layer is coated over the enteric layer, the second release is much less variable, resulting in the specific controlled-release dosage forms according to the invention.

In the field of controlled release dosage systems, the enteric coating is generally used to protect against the acidity of the stomach and is consequently placed over the sustained release layer. In such system, the enteric coating dissolves above a defined pH, subsequently allowing the sustained release layer to act over the entire gut. Therefore, the skilled person would not have designed a composition, in which the sustained release layer is on top of the enteric layer. However, depending on the individual and the previous food-intake, the portion of the gut at which this pH is reached may vary substantially from individual to individual, making the second release of the API much more variable than it is in compositions according to the present invention. Consequently, prior art pharmaceutical compositions are less safe and cannot be applied to such a broad variety of indications as the present dosage form described in more details below.

In one embodiment according to the invention, the core of the solid oral pharmaceutical dosage form, is a pellet and each layer is applied onto the core leading to a coated pellet. The core may be obteind by extrusion or layering of an active ingredient onto a starter core.

These pellet forms provide for several advantages such as improving the efficacy of the dosage form. As the amount of API is divided into smaller units, they are less affected by food intake and lead to higher reproducibility of the pharmacokinetic behaviour.
The pellet form thus further increases the safety of the dosage form as it decreases the variability of the release of the API(s) caused by differences in food-intake between individuals.

Furthermore, there is a lower risk of local irritation through the insoluble membrane and thus a lower risk of dose-dumping through damages of the membrane. Further improvements include a better storability of the pharmaceutical compositions and overall an improved patience compliance.

The coated pellets of the solid oral pharmaceutical dosage form according to the invention can be compressed into a tablet from. The coated pellets can also be encapsulated.

In some embodiments according to the invention, there are one or more additional layers between the layers or on top of the third layer. Such layers can be, for example, protective layers to avoid contact between API and incompatible polymers or to protect the APi from humidity.

Some existing pharmaceutical compositions show similar bi-modal release profiles. Such compositions however are usually a complex multi-particle mixture of beads comprising the API, that are further compressed into a tablet or filled into a capsule, wherein each of the beads shows a different release profile and have to be mixed in a specific ratio in order to show the desired release properties.

Thus, the specific dosage form according to the invention has the advantage to show the highly beneficial release properties without difficult and complex production of multi-particulate beads/pellets with distinct release properties inside a capsule or tablet. This leads to much lower manufacturing variabilities as compared to capsules comprising of a multitude of immediate release and delayed release beads and as such to higher patient safety.

Other tablets exist in prior art that are coated tablets avoiding such manufacturing difficulties. These tablets however, have several other drawbacks, depending on their types and order of layers:
The principle of compositions in which the active ingredient has an enteric coating is that when the tablet reaches a portion with a certain pH in the gut, the coating dissolves and the active ingredient is immediately released. There are two problems with this: the point in the gut where the pH reaches that pH is dependent on each individual and depends on, for example, what the subject has consumed. The release point of the API is therefore not well defined. The second problem is that all of the active ingredient is released at once after the enteric coating is dissolved.

Compositions in which the active ingredient has a sustained release coating shows the problem that the sustained release coating slowly starts becoming permeable from the moment it is put in contact with water. This gradual increase in permeability is entirely pH independent and only depends on the amount of time the tablet has been in contact with water. The problem with a tablet with such a coating is that it slowly starts releasing the active ingredient from the moment the tablet is swallowed.

Furthermore, there are compositions in which the active ingredient has a sustained release coating, which is then itself is coated with an enteric coating. The release of the active ingredient only starts once the tablet has reached a certain pH in the gut. The release is then controlled by the sustained release coating. However, as described above for the tablet with only an enteric coating, the point at which a certain pH is attained is not well defined and there is therefore a lot of variation as to where and when the tablet starts releasing the active ingredient.

These problems are solved with the present invention:
In contrast to the layered compositions of prior art, the active ingredient in the formulation of the present invention is first coated with an enteric coating, producing delayed release (DR) pellets. This enteric coating is then followed by coating with a retard polymer producing a sustained release layer. This produces a tablet with a much better defined release starting point compared to prior art. The reason for this is that the sustained release coating becomes permeable in a time-dependent manner. This means that even if the point with a certain pH is attained early, the enteric coating that is inside the sustained release coating would not be affected until the time when the sustained release coating is sufficiently permeable for the enteric coating to be dissolved. The tablet is therefore able to mitigate the variability of the start of the release caused by the pH variability in the gut.

Therefore, the novel dosage form according to the invention has the advantage that the release of the active ingredient is better controlled and less variable and therefore safer than prior art tablets.

In one embodiment of the present invention the solid oral controlled release dosage form combines both a rapid onset, a delay and sustained plasma concentrations throughout the day or respectively, throughout a desired period. The present dosage form according to the invention therefore, in addition to an enteric coating and a sustained release coating, further comprises a layer comprising an active pharmaceutical ingredient that is coated over the sustained release pellets, allowing for a first rapid release of the API after administration.

This specific order of defined coatings and containing the pharmaceutical active ingredient, allows for a very defined and controlled release of the API. Thus, the novel dosage form is useful for all types of pharmaceutically active ingredients and can extend the duration of action for a desired length of time or provide for a second delayed rapid release of the API after a "gap-phase" in which no release occurs.

The positive effects of the solid oral pharmaceutical dosage form according to the invention are achieved particularly well in case that the enteric polymer is selected from the group comprising esters of cellulose and its derivatives (cellulose acetate phthalate, hydroxypropyl methylcellulose phthalate, hydroxypropyl methylcellulose acetate succinate), polyvinyl acetate phthalate, pH-sensitive methacrylic acid copolymers and shellac.

Most preferred is for the present invention that the enteric polymer is a copolymer derived from esters of acrylic and methacrylic acid.

In the present dosage form, the enteric layer is coated with a sustained release (SR) layer, which ensures the controlled release and less variability from patient to patient in the delayed release phase as detailed above.

To achieve best results, the retard polymer may be selected from the group comprising cellulose derivatives, sodium carboxymethylcellulose, hydoxyalkylcelluloses such as hydroxypropylmethylcellulose and hydroxypropylcellulose, polyethylene oxide, alkylcelluloses such as methylcellulose and ethylcellulose, polyethylene glycol, cellulose acetate, cellulose acetate butyrate, ammonio methacrylate copolymer, polyacrylate dispersion, polyvinylpyrrolidone polyvinyl acetate and mixture thereof.

Most preferred is that the retard polymer is ethylcellulose.

In one embodiment according to the invention, in the solid oral pharmaceutical dosage form, the second sustained release layer is inlayed with one or more pharmaceutically acceptable excipients.

Preferably, the second sustained-release layer comprises one or more pharmaceutically acceptable excipients, which are not enteric coating polymers. Preferably, the second sustained-release layer comprises one or more pH-independent pharmaceutically acceptable excipients.

In the solid oral pharmaceutical dosage form according to the invention, each individual layer may further comprise of excipients like binder, lubricant, plasticizer, pore builder, solvent, or a buffer solution for pH adjustment.

The beneficial bi-modal release profile combined with its high patient compliance and safety, makes the present invention useful for the application of a diverse range of APIs for many indications. Therefore, the solid pharmaceutical dosage form according to the invention may comprise one API or more than one API. The present invention can as such also be applied to deliver multiple drugs with different release profiles. This further increases the range of possible diseases or modalities in which the present invention can be applied.

Thus, in some embodiments, of the dosage form according to the invention, the API in the active core is different to the API in the third layer. In an alternative embodiment, the API in the active core is the same as the API in the third layer.

Therefore, the novel dosage form can be used for APIs, which need to be separated physically due to incompatibility. The dosage form is also appropriate for APIs with different half-lives. In such cases, the API with a longer half-life is in the third immediate release layer of the dosage form, and the API with shorter half-life is in the active core. The dosage form is also appropriate for APIs which are unstable in acid medium. In such embodiments, the sensitive API is contained within the active core.

Preferred APIs according to the invention include but are not limited to opioids, methylphenidate, Zolpidem, Diltiazem, Loratadine, Pseudoephredrin, Fexofenadine, Omeprazol, Diclofenac, or a pharmaceutical acceptable salt thereof.

In case of the API being an opioid analgesic, it can be from the group comprising alfentanil, allylprodine, alphaprodine, anileridine, benzylmorphine, bezitramide, buprenorphine, butorphanol, clonitazene, codeine, besomorphine, dextromoramide, dezocine, diampromide, diamorphone, dihydrocodeine, dihydromorphine, dimenoxadol, Dimepheptanol, dimethylthiambutene, dioxaphetyl butyrate, dipipanone, eptazocine, ethoheptazine, ethylmethylthiambutene, ethylmorphine, etonitazene, fentanyl, heroin, hydrocodone, hydromorphone, hydroxypethidine, isomethadone, ketobemidone, levorphanol, levophenacylmorphane, lofentanil, meperidine, meptazinol, metazocine, methadone, metopone, morphine, myrophine, narceine, nicomorphine, norlevorphanol, normethadone, nalorphine, nalbuphene, normorphine, norpipanone, opium, oxycodone, oxymorphone, papaveretum, pentazocine, phenadoxone, phenomorphane, phenazocine, phenoperidine, piminodine, piritramide, propheptazine, promedol, properidine, propoxyphene, sufentanil, tilidine, and tramadol, hydrocodone, morphine, hydromorphone, oxycodone, buprenorphine, codeine, fentanyl, levorphanol, meperidine, methadone, levomethadone, and dextromethadone.

For treating patients with hypertension and angina pectoris, for example, the dosage form according to the invention may comprise diltiazem, for example diltiazem hydrochloride as the active ingredient. The dosage form of the invention may then comprise diltiazem hydrochloride as API in the immediate-release and prolonged release layer. In one particular embodiment, the dosage form may comprise 300 mg of diltiazem hydrochloride and the active pellets are encapsulated.

In one preferred embodiment of the present invention, the solid oral pharmaceutical dosage form comprises methylphenidate or pharmaceutical acceptable salt, an isomeric form, a prodrug, or metabolite thereof as the active pharmaceutical ingredient. It is preferable that the active ingredient is the hydrochloride salt of methylphenidate. Further preferred is that methylphenidate is a racemic mixture of d- and I-threo enantiomers.

In embodiments, in which the dosage formulation comprises more than one API, possible combinations of APIs are, but not limited to:
- Loratadine PR/Pseudoephedrin IR
- Pseudoephedrin PR/Fexofenadine IR
- Omeprazol ER/Diclofenac PR
- Diclofenac IR/Omeprazol ER

Thus, in some embodiments according to the invention, the dosage form comprises loratadine in the active core and pseudoephedrine hydrochloride in the third immediate-release layer.

The dosage form according to the invention may also comprise immediate-release fexofenadine and sustained-release pseudoephedrine, i.e. fexofenadine in the third layer and pseudoephedrine in the active core. This embodiment is particularly useful for the treatment of allergic rhinitis.

The oral dosage form of the present invention has the advantage to show a bi-modal release profile. Bi-modal release profile according to the invention means that (in case the dosage form comprises only one API) in a first immediate release phase a certain percentage of the active ingredient are released and in second phase, the remaining percentage of the active ingredient.

Thus, in case of methylphenidate or a hydrochloride salt thereof being the active pharmaceutical ingredient, in a first immediate release phase 30-50% methylphenidate is released, and that in a second delayed release the remaining 70-50% of methylphenidate is released.

Preferably 20 to 50% methylphenidate is released in the first immediate release phase, and in the second delayed release, 80 to 50% methylphenidate is released. More preferably in the immediate release 30-50% methylphenidate, and the second delayed release phase 50-70% methylphenidate is released. It is most preferred that 50% methylphenidate is released in the first release phase and 50% during the second release phase.

It was observed that the composition according to the invention, with its specific release profile, was suitable for an administration period of at least twenty hours. Preferably it is used for an administration of more than 24 hours. Accordingly, the present dosage form shows a high level of patient compliance and in one embodiment of the invention, the solid oral pharmaceutical dosage form is administrated once per day.

Alternatively, it can be used for an administration period of less than 20 hours.

A typical dosage form according to the invention comprises 5 - 100 mg methylphenidate or a pharmaceutical acceptable salt thereof. Preferably, it comprises 5-80 mg, 5-70 mg, 10-60 mg, 15-50 mg, 20-40 mg, 25-35 mg, or 30-40 mg. More preferably the dosage form according to the invention comprises about 5, 10, 15, 20, 25, 30, 35, 40, 50, 60, or 70 mg methylphenidate or another pharmaceutical acceptable salt thereof. It is most preferred that the dosage form according to the invention comprises 5, 10, 15, 20, 25, 30, 35, 40, 50, 60, or 70 mg methylphenidate or a pharmaceutical acceptable salt thereof. It is preferred that the pharmaceutical acceptable salt is HCl.

In an embodiment of the invention, in which the API is an opiate, the present dosage from comprises a dosage equivalent to at least 10, 30, 60, 70, 80, 90, 100, 110, 120, 130, 140, 150, 160, 200, 240, 250, 300, 320, or 400 mg of morphine or a pharmaceutical acceptable salt thereof per day. The higher the dosage of morphine equivalent, the more preferred is the embodiment of the dosage form according to the invention. It is preferred that the pharmaceutical acceptable salt is sulfate.

In a more preferred embodiment of the invention, the dosage equivalent ranges from 70 to 300 mg morphine, preferably equivalent to 75 to 270 mg morphine, more preferably 85 to 260 mg morphine and most preferably a dosage equivalent to 80 to 250 mg morphine per day. Preferably, morphine sulfate is used in said ranges.

In certain preferred embodiments, the oral dosage form provides a time to maximum plasma concentration at about 0.5 to about 4 hours after oral administration under fastening conditions. In certain further preferred embodiments, the duration of effect provided by the methylphenidate contained in the oral dosage form falls below effective plasma concentrations at about 8 to about 12 hours after oral administration.

The novel solid oral pharmaceutical dosage form may also additionally comprise compounds for abuse prevention. Such compounds may be selected from the group of compounds comprising viscosity increasing agents, emetic, irritative substances, and/or antagonists of the active ingredient.

As noted before, the present pharmaceutical dosage form may be indicated in a broad spectrum of diseases and treatments due to its defined controlled release with little variability and improved safety. For example, the dosage form may be used, in the treatment of Attention Deficit Hyperactivity Disorder (ADHD), diabetes, pain, insomnia, high blood pressure, allergies such as allergic rhinitis, gastroesophageal reflux diseases, and many others.

Due to the high patient compliance and safety, the present invention provides for a new oral dosage form which represents a great improvement over currently available preparations available for conditions such as Attention Deficit Hyperactivity Disorder (ADHD). Thus, in one embodiment, the dosage from according to the invention is used in the treatment of Attention Deficit Hyperactivity Disorder (ADHD). In such cases, the new oral dosage form may comprise methylphenidate or similarly acting drugs and ensures adequate treatment of ADHD throughout a child's school day. The new oral dosage form, in such cases may allow a child with ADHD, to be maximally treated throughout the daytime, while being administered only once, i.e., in the morning.

### EXAMPLES

### Example 1

### Production of modified release bi-modal release compositions comprising Methylphenidate

### Step (i): Preparation of active pellets

An active pharmaceutical ingredient (API) spraying suspension was prepared in the first stage. 0.2kg Hypromellose and 2.0kg methylphenidate hydrochloride were dissolved in 12.4kg water. 0.2kg talc was suspended in this solution. Resulting suspension was stirred during the spraying process.

In the second stage, 0.89kg Suglets^{®} pellets were filled into a fluid-bed processor. The suspension prepared in the first stage was sprayed onto the pellets, and the resulting active pellets were sieved.

### Step (ii): Preparation of delayed release pellets

A delayed release pellet coating suspension was prepared in the first stage. 8.5kg Methacrylic Acid copolymer was added to 31.0kg water and stirred.
4.25kg triethyl citrate and 4.25kg talc were added to 15.5kg water and homogenized. The suspension was added under stirring to Eudragit^{®} dispersion. The resulting suspension is stirred during spraying.
In the second stage, active pellets from step (i) were filled into the fluid-bed processor. The delayed release pellet coating suspension was then sprayed onto the active pellets. Resulting delayed release pellets were sieved.

### Step (iii): Preparation of sustained release pellets

0.84 kg ethylcellulose and 0.56 kg hydroxypropylcellulose were added to a solvent mixture of 31.0kg of isopropyl alcohol and 1.0 kg water, under stirring in the first stage. 0.17 kg triethyl citrate was added under stirring until a clear solution was obtained. 0.25 kg talc was also added under stirring to produce sustained release pellet coating suspension.
In the second stage, delayed release pellets prepared in step (ii), were filled into the fluid-bed processor. The sustained release pellet coating suspension was sprayed onto the delayed release pellets under stirring. Resulting sustained release pellets were sieved.

### Step (iv): Preparation of active coated pellets

2.0kg Methylphenidate HCl and 0.2kg Hypromellose were dissolved in 13.0kg water under stirring in the first stage to produce active pellet coating solution.
In the second stage, sustained release pellets from step (iii) were filled into the fluid-bed processor. The active pellet coating solution was sprayed onto the sustained release pellets. Resulting active coated pellets were sieved.
In the third stage, the active coated pellets were blended with 0.12kg talc and the resulting mixture was filled into hard gelatine capsules. The capsules were finally packed in HDPE bottles.

### Example 2

### Preparation of modified release bi-modal release compositions comprising Methylphenidate

### Step (i): Preparation of active pellets

Methylphenidate hydrochloride spraying suspension was prepared by dissolving 0.5kg Hypromellose and 5.0kg methylphenidate hydrochloride in 31.0kg water, followed by addition of 0.5kg talc. 12.6kg Suglets^{®} pellets in a fluid-bed processor were sprayed with methylphenidate hydrochloride spraying suspension.

### Step (ii): Preparation of delayed release pellets

A delayed release pellet coating suspension was prepared by adding 45.0kg Methacrylic Acid Copolymer to a mixture of isopropyl alcohol and water followed by addition of 4.5kg triethyl citrate and 22.5kg talc. Active pellets from step (i) were filled into the fluid-bed processor, and the delayed release pellet coating suspension was then sprayed onto it.

### Step (iii): Preparation of sustained release pellets

A sustained release pellet coating suspension was prepared by adding 1.25kg ethylcellulose and 1.25kg hydroxypropylcellulose to a solvent mixture of 46kg of isopropyl alcohol and 1.6kg water, followed by addition of 0.25kg triethyl citrate, and 0.37kg talc. The said suspension was sprayed on to the delayed release pellets prepared in step (ii) in a fluid-bed processor.

### Step (iv): Preparation of active coated pellets

5.0kg Methylphenidate HCl and 0.5kg Hypromellose were dissolved in 32.5kg water to produce active pellet coating solution. The said solution was sprayed onto the sustained release pellets from step (iii) in a fluid-bed processor. The active coated pellets were finally blended with 0.5kg talc and encapsulated.

### Example 3

### Preparation of modified release bi-modal release compositions of Zolpidem

### Step (i): Preparation of active pellets

Zolpidem tartrate spraying suspension was prepared by dissolving 22.3g potassium hydrogen tartrate, hypromellose 4.3g and 22.3g zolpidem tartrate in a mixture of 186.0g ethanol and 46.0g water, followed by addition of 2.1g talc. 100.0g Cellets^{®} pellets in a fluid-bed processor were sprayed with zolpidem tartrate spraying suspension to produce active pellets.

### Step (ii): Preparation of delayed release pellets

A delayed release pellet coating suspension was prepared by adding 64.8g Eudragit L100^{®} to a mixture of 355.0g water and 36.0g 1.0N aqueous ammonia solution, followed by addition of 32.4g triethyl citrate and 32.4g talc. Active pellets from step (i) were filled into the fluid-bed processor, and the delayed release pellet coating suspension was then sprayed onto it.

### Step (iii): Preparation of sustained release pellets

A sustained release pellet coating suspension was prepared by adding 4.7g Kollicoat^{®} SR 30 D and 1.4g hydroxypropylcellulose to 8.2g water, followed by addition of 0.14g triethyl citrate, and 0.56g talc. The said suspension was sprayed on to the delayed release pellets prepared in step (ii) in a fluid-bed processor.

### Step (iv): Preparation of active coated pellets

18.8g zolpidem tartrate, 18.8g potassium hydrogen tartrate and 3.6g Hypromellose were dissolved in a mixture of 156.0g ethanol and 37.0g water to produce active pellet coating solution. The said solution was sprayed onto the sustained release pellets from step (iii) in a fluid-bed processor. The active coated pellets were finally blended with 1.7g talc.

## Claims

1. A solid oral pharmaceutical dosage form comprising at least one active pharmaceutical ingredient or a pharmaceutical acceptable salt, an isomeric form, a prodrug, or metabolite thereof, wherein the solid oral pharmaceutical dosage form comprises:
(i) an immediate release active core
(ii) a first delayed-release layer comprising an enteric coating
(iii) a second sustained release layer comprising a retard polymer, and
(iv) a third immediate release layer comprising an active pharmaceutical ingredient
wherein the solid oral pharmaceutical dosage form exhibits a bi-modal release profile of the at least one active pharmaceutical ingredient, and wherein the active pharmaceutical ingredient in the active core is the same or different than the active pharmaceutical ingredient in the third layer.

2. A solid oral pharmaceutical dosage form according to claim 1, wherein the core is a pellet and each layer is applied onto the core leading to a coated pellet.

3. A solid oral pharmaceutical dosage form according to claim 2, wherein the coated pellets are compressed into a tablet form.

4. A solid oral pharmaceutical dosage form according to claims 2, wherein the pellets are encapsulated.

5. A solid oral pharmaceutical dosage form according to any of the previous claims, wherein there are one or more additional layers between the layers or on top of the third layer.

6. A solid oral pharmaceutical dosage form according to any of the previous claims, wherein the first delayed-release layer comprises an enteric polymer selected from the group comprising esters of cellulose and its derivatives (cellulose acetate phthalate, hydroxypropyl methylcellulose phthalate, hydroxypropyl methylcellulose acetate succinate), polyvinyl acetate phthalate, pH-sensitive methacrylic acid copolymers and shellac.

7. A solid oral pharmaceutical dosage form according to any of the previous claims, wherein the second sustained-release layer comprises one or more pH-independent pharmaceutically acceptable excipients.

8. A solid oral pharmaceutical dosage form according to any of the preceding claims, wherein the retard polymer is selected from the group comprising cellulose derivatives, sodium carboxymethylcellulose, hydoxyalkylcelluloses such as hydroxypropylmethylcellulose and hydroxypropylcellulose, polyethylene oxide, alkylcelluloses such as methylcellulose and ethylcellulose, polyethylene glycol, cellulose acetate, cellulose acetate butyrate, ammonio methacrylate copolymer, polyacrylate dispersion, polyvinylpyrrolidone polyvinyl acetate and mixture thereof.

9. A solid oral pharmaceutical dosage form according to any of the preceding claims, wherein each individual layer may further comprise of excipients like binder, lubricant, plasticizer, pore builder, solvent, or a buffer solution for pH adjustment.

10. A solid oral pharmaceutical dosage form according to any of the preceding claims, wherein bi-modal release profile means an immediate release of 30-50% of the active pharmaceutical ingredient, and a second delayed release of the remaining 70-50% of the active pharmaceutical ingredient.

11. A solid oral pharmaceutical dosage form according to any of the preceding claims, wherein the dosage form is administered once per day.

12. A solid oral pharmaceutical dosage form according to any of the preceding claims, additionally comprising compounds for abuse prevention.

13. A solid oral pharmaceutical dosage form according to any of the preceding claims for use in the treatment of a disease or modality selected from the group comprising Attention Deficit Hyperactivity Disorder (ADHD), diabetes, pain, insomnia, high blood pressure, allergies such as allergic rhinitis, and gastroesophageal reflux diseases.

14. A solid oral pharmaceutical dosage form according to any of the previous claims, wherein the at least one active pharmaceutical ingredient is methylphenidate or a pharmaceutical acceptable salt thereof, wherein the methylphenidate is a racemic mixture of d-and I-threo enantiomers or the d-threo enantiomer of methylphenidate or a pharmaceutical salt thereof.

15. A solid oral pharmaceutical dosage form according to any of the preceding claims, wherein the dosage form comprises 5 -100 mg methylphenidate or the hydrochloride salt thereof.
